# EUROPEAN PATENT APPLICATION

(11) **EP 1 415 985 A1**
(43) Date of publication of application: **06.05.2004**
(21) Application number: 02758805.2
(22) Date of filing: 08.08.2002
(51) Int. Cl.: C07D 209/48, C07D 205/04, C07C 229/08, C07C 227/18

(54) **PROCESS FOR PRODUCING OPTICALLY ACTIVE AZETIDINE-2-CARBOXYLIC ACID**

(30) Priority: 08.08.2001 JP 2001240673
(71) Applicant: KANEKA CORPORATION, Osaka-shi, Osaka 530-8288 (JP)
(72) Inventor: KONDO, Takeshi, Kakogawa-shi, Hyogo 675-0015 (JP); UEYAMA, Noboru, Kobe-shi, Hyogo 651-1211 (JP)
(74) Representative: Hubert, Philippe
(86) International application number: PCT/JP2002/008097
(87) International publication number: WO 2003/014081

(57) **Abstract**

The present invention provides an efficient, simple, and commercially advantageous process for producing optically active azetidine-2-carboxylic acid, which is an important material for medicines.

The process includes the steps of halogenating an optically active N-protected 4-amino-2-hydroxybutyric acid following inversion of the configuration to produce an optically active N-protected 4-amino-2-halobutyryl halide; hydrolyzing the halide; deprotecting the amino group of the hydrolyzed product to produce an optically active 4-amino-2-halobutyric acid; cyclizing the product in an alkaline aqueous solution; and then protecting the amino group of the cyclized product to produce an optically active N-protected azetidine-2-carboxylic acid.

The present invention also provides an optically active N-protected 4-amino-2-halobutyryl halide represented by general formula (2): 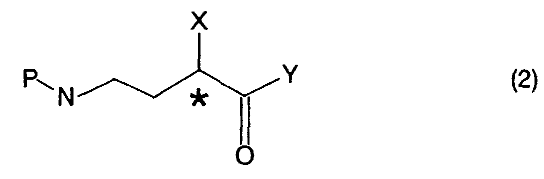 (wherein P represents a protective group for the primary amino group; * indicates that the carbon atom is asymmetric; and each of X and Y independently represents a halogen atom), which is useful for producing the optically active azetidine-2-carboxylic acid.

## Description

### Technical Field

The present invention relates to a process for producing optically active azetidine-2-carboxylic acid, which is an important material for medicines, and to a useful intermediate thereof.

### Background Art

In general, the following processes for producing optically active azetidine-2-carboxylic acid derivatives are well known:
(1) L-2,4-diaminobutyric acid is allowed to react with hydrochloric acid and nitrous acid to produce L-4-amino-2-chlorobutyric acid. The L-4-amino-2-chlorobutyric acid is mixed with an aqueous barium hydroxide solution and the mixture is heated to produce D-azetidine-2-carboxylic acid (Biochemical Journal, Vol. 64, p. 323 (1956)).
(2) γ-Butyrolactone is allowed to react with bromine in the presence of red phosphorus. The resultant product is allowed to react with benzyl alcohol saturated with hydrogen chloride gas to produce DL-2,4-dibromobutyric acid benzyl ester. The product is allowed to react with benzhydrylamine to produce DL-N-diphenylmethylazetidine-2-carboxylic acid benzyl ester. The product is reduced with hydrogen in the presence of palladium carbon in methanol to produce DL-azetidine-2-carboxylic acid. The DL-azetidine-2-carboxylic acid is allowed to react with benzyloxycarbonyl chloride to produce DL-N-(benzyloxycarbonyl)azetidine-2-carboxylic acid. The DL-N-(benzyloxycarbonyl)azetidine-2-carboxylic acid is optically resolved with L-tyrosine hydrazide to produce L-N-(benzyloxycarbonyl)azetidine-2-carboxylic acid. Finally, the L-N-(benzyloxycarbonyl)azetidine-2-carboxylic acid is reduced again with hydrogen in the presence of palladium carbon in methanol to produce L-azetidine-2-carboxylic acid (Journal of Heterocyclic Chemistry, Vol. 6, pp. 435 and 993 (1969)).
(3) L-N-(tosyl)methionine is subjected to S-alkylation to produce L-N-(tosyl)methionine sulfonium salt. The product is mixed with an aqueous sodium hydroxide solution and the mixture is heated to produce L-N-tosyl-α-amino-γ-butyrolactone. The L-N-tosyl-α-amino-γ-butyrolactone is allowed to react with gaseous hydrogen halide in alcohol to produce alkyl L-N-tosyl-2-amino-4-halobutyrate. The product is cyclized with sodium hydride in dimethylformamide to produce L-N-(tosyl)azetidine-2-carboxylic acid. The L-N-(tosyl)azetidine-2-carboxylic acid is treated with metallic sodium in liquid ammonia in order to deprotect the tosyl group. Thus, L-azetidine-2-carboxylic acid is produced (Chemistry Letters, p. 5 (1973)).
(4) L-aspartic acid diester is cyclized to produce a 4-oxo-2-azetidine carboxylic acid derivative. The product is reduced with lithium aluminum hydride to produce azetidine-2-methanol. This product is subjected to N-tert-butoxycarbonylation to produce N-(tert-butoxycarbonyl)azetidine-2-methanol. The N-(tert-butoxycarbonyl)azetidine-2-methanol is oxidized to produce N-(tert-butoxycarbonyl)azetidine-2-carboxylic acid (PCT Publication No. WO9847867).
(5) A racemic disubstituted butyrate is allowed to react with an optically active alkylbenzylamine to produce a diastereoisomeric pair of optically active N-(alkylbenzyl)azetidine-2-carboxylic acid ester. The ester group is then hydrolyzed to produce a diastereoisomeric pair of optically active N-(alkylbenzyl)azetidine-2-carboxylic acid (Japanese Unexamined Patent Application Publication No. 10-130231).
(6) An N-substituted azetidine-2-carboxylic acid ester is treated with an enzyme that displays enantioselective hydrolyzability to produce a mixture of the optically active N-substituted azetidine-2-carboxylic acid and the optically active N-substituted azetidine-2-carboxylic acid ester. The mixture is then separated (Japanese Unexamined Patent Application Publication No. 11-46784).
(7) Racemic N-acylazetidine-2-carboxylic acid ester is hydrolyzed with an enzyme that displays enantioselectivity to produce a mixture of optically active N-acylazetidine-2-carboxylic acid and optically active N-acylazetidine-2-carboxylic acid ester. The mixture is then separated (PCT Publication No. WO9802568).
(8) An optically active N-substituted α-amino-γ-halobutyric acid ester that is derived from optically active methionine is cyclized to produce the optically active N-substituted azetidine-2-carboxylic acid ester. The ester group is then hydrolyzed to produce the optically active N-substituted azetidine-2-carboxylic acid (Japanese Unexamined Patent Application Publication No. 10-120648).
(9) In this process, 4-amino-2-halobutyric acid is produced by esterifying optically active 4-amino-2-hydroxybutyric acid, followed by halogenation, cyclization, and hydrolysis of the product. The 4-amino-2-halobutyric acid is then cyclized to produce L-azetidine-2-carboxylic acid. Alternatively, 4-amino-2-halobutyric acid is produced by esterifying optically active 4-amino-2-hydroxybutyric acid, halogenating the product, allowing the product to react with sulfuric acid, (furthermore, allowing the product to desalt), and hydrolyzing the product. The 4-amino-2-halobutyric acid is then cyclized to produce L-azetidine-2-carboxylic acid (PCT Publication No. WO0069817).

Unfortunately, the processes described above have the following disadvantages.
In process (1), L-2,4-diaminobutyric acid is expensive. Furthermore, more expensive D-2,4-diaminobutyric acid is required to produce more useful L-azetidine-2-carboxylic acid. In addition, since conditions such as the reaction temperature and reaction time in the first step influence the optical purity of the target compound, the reaction must be strictly optimized.
Process (2) takes many steps to implement, in addition, benzhydrylamine is expensive. Furthermore, the undesired optically active substance produced by optical resolution is disposed of, as long as a beneficial racemizing process is not developed. Thus, this process is economically disadvantageous.
Process (3) takes many steps to implement, in addition, a cryogenic device that requires careful handling is necessary, because metallic sodium must be treated in liquid ammonia in the step of deprotecting the tosyl group.
In process (4), lithium aluminum hydride, which requires careful handling, is used as a reductant of azetidinone. Accordingly, this process is disadvantageous to industrial production.
In process (5), stereoselectivity in the reaction is generally not high and the diastereoisomeric pair of optically active N-(alkylbenzyl)azetidine-2-carboxylic acid ester includes a large amount of undesired stereoisomer. Accordingly, in order to produce the desired stereoisomer, the large amount of undesired stereoisomer must be separated. Thus, this process is not advantageous in terms of reaction efficiency and economical efficiency, and is disadvantageous to industrial production.
In processes (6) and (7), both of the processes utilize an enzyme to optically resolve the stereoisomer. Therefore, the theoretical yield to recover the desired stereoisomer is less than 50% and a large amount of undesired stereoisomer must be separated. Thus, this process is not advantageous in terms of reaction efficiency and economical efficiency, and is disadvantageous to industrial production.
In process (8), a compound having an ester group is cyclized in order to synthesize the optically active N-substituted azetidine-2-carboxylic acid. Accordingly, this process requires a step of hydrolyzing the ester group of the optically active N-substituted azetidine-2-carboxylic acid ester.
Process (9) takes many steps to derive the 4-amino-2-halobutyric acid from optically active 4-amino-2-hydroxybutyric acid. Furthermore, the resultant N-protected L-azetidine-2-carboxylic acid does not have a high optical purity. Thus, this process is not advantageous in terms of reaction efficiency and economical efficiency, and is disadvantageous to industrial production.

### Summary of the Invention

As described above, each of the known processes includes problems to be solved in terms of commercial manufacturing process. In view of the above current status, it is an object of the present invention to provide an efficient, economical, and commercially preferable process for manufacturing azetidine-2-carboxylic acid.

As a result of intensive study for solving the problems, the present inventors have successfully accomplished the present invention.

The present invention provides a process for producing an optically active N-protected 4-amino-2-halobutyryl halide represented by general formula (2): 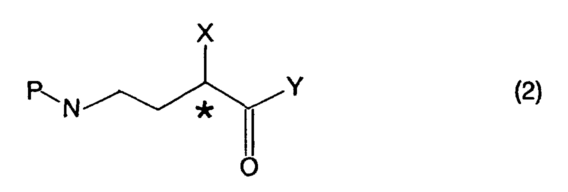 (wherein P represents a protective group for the primary amino group; * indicates that the carbon atom is asymmetric; and each of X and Y independently represents a halogen atom), the process including halogenating an optically active N-protected 4-amino-2-hydroxybutyric acid following inversion of the configuration, the optically active N-protected 4-amino-2-hydroxybutyric acid being represented by general formula (1): 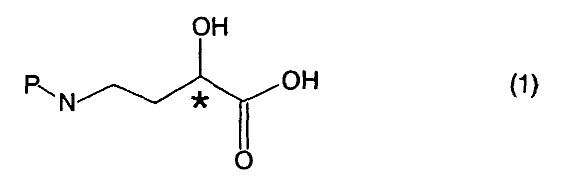 (wherein P and * are as defined above).

The present invention provides a process for producing an optically active N-protected 4-amino-2-halobutyric acid represented by general formula (3): 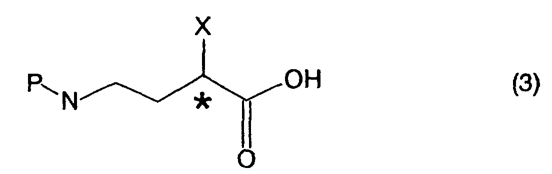 (wherein P represents a protective group for the primary amino group; * indicates that the carbon atom is asymmetric; and X represents a halogen atom), the process including hydrolyzing an optically active N-protected 4-amino-2-halobutyryl halide represented by general formula (2).

The present invention provides a process for producing an optically active N-protected 4-amino-2-halobutyric acid represented by general formula (3), the process including the steps of halogenating an optically active N-protected 4-amino-2-hydroxybutyric acid represented by general formula (1) following inversion of the configuration to produce an optically active N-protected 4-amino-2-halobutyryl halide represented by general formula (2); and hydrolyzing the optically active N-protected 4-amino-2-halobutyryl halide represented by general formula (2).

The present invention provides a process for producing an optically active azetidine-2-carboxylic acid represented by general formula (4): 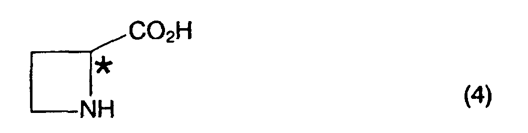 (wherein * indicates that the carbon atom is asymmetric), the process including the steps of halogenating an optically active N-protected 4-amino-2-hydroxybutyric acid represented by general formula (1) following inversion of the configuration to produce an optically active N-protected 4-amino-2-halobutyryl halide represented by general formula (2); hydrolyzing the optically active N-protected 4-amino-2-halobutyryl halide represented by general formula (2) to produce an optically active N-protected 4-amino-2-halobutyric acid represented by general formula (3); deprotecting the amino group of the optically active N-protected 4-amino-2-halobutyric acid represented by general formula (3); and cyclizing the deprotected product in an alkaline aqueous solution.

Furthermore, the present invention provides a process for producing an optically active N-protected azetidine-2-carboxylic acid represented by general formula (5): 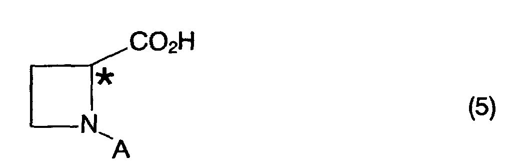 (wherein A represents a protective group for the secondary amino group; and * indicates that the carbon atom is asymmetric), the process including the steps of halogenating an optically active N-protected 4-amino-2-hydroxybutyric acid represented by general formula (1) following inversion of the configuration, to produce an optically active N-protected 4-amino-2-halobutyryl halide represented by general formula (2); hydrolyzing the optically active N-protected 4-amino-2-halobutyryl halide represented by general formula (2) to produce an optically active N-protected 4-amino-2-halobutyric acid represented by general formula (3); deprotecting the amino group of the optically active N-protected 4-amino-2-halobutyric acid represented by general formula (3); cyclizing the deprotected product in an alkaline aqueous solution to produce an optically active azetidine-2-carboxylic acid represented by general formula (4); and reacting the optically active azetidine-2-carboxylic acid represented by general formula (4) with an amino group-protecting agent.

The present invention also provides an optically active N-protected 4-amino-2-halobutyryl halide, which is a new compound, represented by general formula (2).

### Detailed Disclosure of Invention

The present invention will now be described in detail.

In this specification, an NH₂ group bonding to one atom other than a hydrogen atom is defined as a primary amino group, an NH group bonding to two atoms other than hydrogen atoms is defined as a secondary amino group, an N group bonding to three atoms other than hydrogen atoms is defined as a tertiary amino group, and an N+ group bonding to four atoms including hydrogen atom is defined as a quaternary amino group. If a nitrogen atom includes an unsaturated bond, each bond is counted as one atom. For example, pyridine is defined as a compound having a tertiary amino group.

An optically active N-protected 4-amino-2-hydroxybutyric acid represented by general formula (1) is synthesized, for example, as follows: L-glutamic acid is allowed to react with nitrous acid to form a cyclic lactone, and the cyclic lactone ring is opened with ammonia to form a monoamide. Then the monoamide is degraded with antiformin by Hoffman degradation to form L-4-amino-2-hydroxybutyric acid (Japanese Unexamined Patent Application Publication No. 50-4019). Finally, N-protection to the L-4-amino-2-hydroxybutyric acid is performed by a known process.

The optically active N-protected 4-amino-2-hydroxybutyric acid (1) is halogenated following inversion of the configuration at the second position so as to produce an optically active N-protected 4-amino-2-halobutyryl halide represented by general formula (2).

In this specification, "inversion of the configuration" indicates that (R) compounds are converted to (S) compounds, or (S) compounds are converted to (R) compounds.

In general formulas (1) and (2), P represents a protective group for the primary amino group. The protective group protects the amino group during the reactions of the present invention. Examples of the protective group are disclosed in "PROTECTIVE GROUPS IN ORGANIC SYNTHESIS, second edition" (JOHN WILEY & SONS 1991). In terms of handling and cost, the protective group preferably includes a phthalimido group and alkoxy carbonyl groups such as a benzyloxycarbonyl group, tert-butoxycarbonyl group, methoxycarbonyl group, and ethoxycarbonyl group.

In general formula (2), X and Y independently represent a halogen atom, such as chlorine, bromine, iodine, or fluorine. In terms of the reactivity in the subsequent steps and preventing racemization, chlorine and bromine are particularly preferable.

The optically active N-protected 4-amino-2-halobutyryl halide represented by general formula (2) is a useful new compound developed by the present inventors for producing optically active azetidine-2-carboxylic acid derivatives (5), which are important materials for medicines.

According to a halogenation step of the present invention, the optically active N-protected 4-amino-2-hydroxybutyric acid (1) is allowed to react with a halogenating agent. Examples of the halogenating agent include a fluorinating agent such as hydrofluoric acid-potassium fluoride; a chlorinating agent such as thionyl chloride, phosphorus trichloride, phosphorus pentachloride, hydrochloric acid, phosphorus oxychloride, and triphenylphosphine-carbon tetrachloride; a brominating agent such as thionyl bromide, thionyl chloride-hydrobromic acid, phosphorus tribromide, hydrobromic acid, and triphenylphosphine-carbon tetrabromide; and a iodinating agent such as hydroiodic acid, triphenylphosphine-iodine, and trimethylchlorosilane-sodium iodide. In terms of handling and stereoselectivity, thionyl chloride, thionyl bromide, and thionyl chloride-hydrobromic acid are preferably used, and thionyl chloride is more preferably used.

The content of the halogenating agent may be one molar equivalent or more of the optically active N-protected 4-amino-2-hydroxybutyric acid (1). Generally, in terms of economical efficiency, preferably, ten molar equivalents or less, more preferably, five molar equivalents or less, and most preferably, two molar equivalents or less of the optically active N-protected 4-amino-2-hydroxybutyric acid (1) are used in the halogenation step.

Any reaction solvent that does not inhibit the halogenation step may be used. Examples of the solvents include aliphatic hydrocarbons such as pentane, hexane, heptane, cyclohexane, and petroleum ether; esters such as ethyl acetate, methyl acetate, propyl acetate, and methyl propionate; aromatic hydrocarbons such as toluene, benzene, and xylene; nitriles such as acetonitrile and propionitrile; ethers such as tert-butylmethylether, diethyl ether, ethylene glycol dimethyl ether, diisopropyl ether, tetrahydrofuran, and dioxane; ketones such as acetone and ethyl methyl ketone; amides such as N,N-dimethylformamide, N,N-dimethylacetamide; sulfoxides such as dimethylsulfoxide; halogenated hydrocarbons such as methylene chloride, chloroform, and carbon tetrachloride; and thionyl chloride. These solvents may be used alone or in combination.

When thionyl chloride is used as the reaction solvent at an amount of one molar equivalent or more of the amount of the optically active N-protected 4-amino-2-hydroxybutyric acid (1), the halogenating agent need not be added.

In terms of the solubility of the optically active N-protected 4-amino-2-hydroxybutyric acid (1) and the stability to the halogenating agent, preferable solvents include dioxane, tetrahydrofuran, ethylene glycol dimethyl ether, ethyl acetate, toluene, thionyl chloride, and a mixture thereof. The ratio of the solvents in the mixture is not limited.

The concentration of the optically active N-protected 4-amino-2-hydroxybutyric acid (1) depends on the kind of reaction solvent used. In general, in order to achieve high reaction efficiency, the concentration of the optically active N-protected 4-amino-2-hydroxybutyric acid (1) is preferably 1 percent by weight or more, and more preferably, 5 percent by weight or more. Furthermore, in order to achieve high reaction efficiency, the concentration of the optically active N-protected 4-amino-2-hydroxybutyric acid (1) is preferably 50 percent by weight or less, and more preferably, 30 percent by weight or less.

Although the reaction temperature during the halogenation step depends on the kind of halogenating agent and the kind of reaction solvent used, the reaction temperature generally ranges from the solidifying point to the boiling point of the reaction solvent. In order to complete the reaction in a short time, a high reaction temperature is preferable, whereas in order to prevent racemization, a low reaction temperature is preferable. The reaction temperature is preferably 10°C or more, and more preferably, 20°C or more. The reaction temperature is preferably 100°C or less, and more preferably, 60°C or less.

The reaction time of the halogenation step depends on the kind of halogenating agent, the kind of reaction solvent, and the reaction temperature used. When the halogenation is performed at a temperature ranging from 20°C to 60°C, the reaction time is, in general, 1 hour to 24 hours.

In the halogenation step, adding a compound having a primary amino group, a compound having a secondary amino group, a compound having a tertiary amino group, or a compound having a quaternary amino group is effective at improving the yield. Examples of the additives include ammonia salts, amines, imines, amides, imides, urea, and salts thereof.

Specifically, examples of the additives include aliphatic amines such as triethylamine, N,N-diisopropylethylamine, N-methylmorpholine, diisopropylamine, butylamine, benzylamine, phenethylamine, alanine, glutamine, and γ-aminobutyric acid (GABA) esters; aromatic amines such as pyridine, picoline, lutidine, quinoline, isoquinoline, and imidazole; amines bonding to an aromatic ring such as aniline, and N,N-dimethylaniline; amides such as N,N-dimethylformamide, N,N-dimethylacetamide; and amine salts such as benzyltriethylammonium chloride, tetrabutylammonium bromide, and carnitine. In view of availability and economical efficiency, for example, pyridine, triethylamine, imidazole, N,N-dimethylformamide, or benzyltriethylammonium chloride is preferably used.

The additive content is not limited in the present invention. In general, the additive content is preferably 0.01 mole percent or more of the optically active N-protected 4-amino-2-hydroxybutyric acid (1). In terms of economical efficiency, the additive content is more preferably 0.1 mole percent of the optically active N-protected 4-amino-2-hydroxybutyric acid (1). Furthermore, the additive content is preferably 100 mole percent or less of the optically active N-protected 4-amino-2-hydroxybutyric acid (1). In terms of economical efficiency, the additive content is more preferably 20 mole percent or less, and most preferably, 10 mole percent or less of the optically active N-protected 4-amino-2-hydroxybutyric acid (1).

When a volatile halogenating agent such as thionyl chloride is used in the halogenation step, the halogenation is performed as described above and then the reaction solvent is removed under reduced pressure. Thus, the optically active N-protected 4-amino-2-halobutyryl halide (2) is produced. In this case, the resultant mixture is used in the subsequent step without further treatment.

In the subsequent step, the optically active N-protected 4-amino-2-halobutyryl halide (2) is hydrolyzed to produce an optically active N-protected 4-amino-2-halobutyric acid (3).

The solvent used in the hydrolysis step includes water or a mixed solvent of water and an organic solvent. The organic solvent is not limited and the solvents described in the halogenation step may be used. In terms of simplifying the operation, after the halogenation step, water is directly added to the reaction mixture. In general, although adding water allows the hydrolysis to be rapidly completed, an acid such as hydrochloric acid or a base such as sodium hydroxide may be added to the reaction mixture.

The reaction temperature during the hydrolysis depends on the kind of reaction solvent used, the reaction temperature generally ranges from the solidifying point to the boiling point of the reaction solvent. In order to complete the reaction in a short time, a high reaction temperature is preferable, whereas in order to prevent racemization, a low reaction temperature is preferable. In general, the reaction temperature is preferably 0°C or more. In general, the reaction temperature is preferably 100°C or less, and more preferably, 30°C or less.

After the completion of the hydrolysis, when the subsequent step (i.e., deprotection of the primary amino group) is performed in the aqueous system, the reaction mixture is used in the subsequent step as it is or after neutralization. Alternatively, the resultant optically active N-protected 4-amino-2-halobutyric acid (3) may be isolated by a common method, for example, extraction or chromatography.

Then, the primary amino group of the optically active N-protected 4-amino-2-halobutyric acid (3) is deprotected to produce optically active 4-amino-2-halobutyric acid. The process for deprotecting the primary amino group is disclosed in, for example, "PROTECTIVE GROUPS IN ORGANIC SYNTHESIS, second edition" (JOHN WILEY & SONS 1991). According to this process, the N-protected compound is allowed to react first with hydrazine hydrate and then with an acid such as sulfuric acid, thereby deprotecting the amino group. The subsequent step includes cyclization in an alkaline aqueous solution. Accordingly, when the deprotection is performed in an aqueous solution or in a water-miscible organic solvent, the reaction mixture may be used in the subsequent step as it is. The resultant optically active 4-amino-2-halobutyric acid may be isolated by a common method, for example, extraction or chromatography. Before the subsequent step, the pH of the resultant aqueous solution is preferably adjusted to be neutral with, for example, an aqueous sodium hydroxide solution.

In the subsequent step, the optically active 4-amino-2-halobutyric acid is cyclized in an alkaline aqueous solution to produce optically active azetidine-2-carboxylic acid represented by general formula (4).

Examples of the bases used for the alkaline aqueous solution include alkali metal bases such as sodium hydroxide, cesium hydroxide, potassium hydroxide, lithium hydroxide, and cesium carbonate; alkali earth metal bases such as barium hydroxide, calcium hydroxide, magnesium hydroxide, and magnesium oxide. Sodium hydroxide, barium hydroxide, magnesium hydroxide, and magnesium oxide are preferably used.

Although the base content is not limited, the base content is preferably one molar equivalent or more of the optically active 4-amino-2-halobutyric acid. Furthermore, the base content is preferably 30 molar equivalents or less, and more preferably, 10 molar equivalents or less of the optically active 4-amino-2-halobutyric acid aqueous solution.

In terms of economic efficiency, the concentration of the optically active 4-amino-2-halobutyric acid in the cyclization step is preferably 1 percent by weight or more, and more preferably, 2 percent by weight or more. Furthermore, in terms of improving the yield, the concentration of the optically active 4-amino-2-halobutyric acid is preferably 50 percent by weight or less, and more preferably, 30 percent by weight or less.

Although the reaction temperature during the cyclization step depends on the kind of base used, the reaction temperature generally ranges from the solidifying point to the boiling point of water, which is a reaction solvent. In order to complete the reaction in a short time, a high reaction temperature is preferable, whereas in order to prevent racemization, a low reaction temperature is preferable. The reaction temperature is preferably 30°C or more, and more preferably, 50°C or more. Furthermore, the reaction temperature is preferably 100°C or less.

The reaction time of the cyclization step depends on the kind and the content of the base, and the reaction temperature. When the cyclization is performed at a temperature ranging from 70°C to 100°C, the reaction time is generally about 20 minutes to 12 hours.

After the completion of the cyclization step, the reaction mixture is used in the subsequent step of protecting the amino group as it is or after neutralization. If necessary, the reaction mixture may be purified by, for example, ion-exchange chromatography to isolate optically active azetidine-2-carboxylic acid (4).

In the subsequent step, the optically active azetidine-2-carboxylic acid (4) is allowed to react with an amino group-protecting agent to produce an optically active N-protected azetidine-2-carboxylic acid represented by general formula (5).

In general formula (5), A represents a protective group for the secondary amino group. The protective group protects the amino group during the reaction of the present invention. Examples of the protective group are disclosed in "PROTECTIVE GROUPS IN ORGANIC SYNTHESIS, second edition" (JOHN WILEY & SONS 1991). Specifically, examples of the protective group include alkoxy carbonyl protective groups such as a tert-butoxycarbonyl group, benzyloxycarbonyl group, allyloxycarbonyl group, methoxycarbonyl group, and ethoxycarbonyl group; acyl protective groups such as a benzoyl group, acetyl group, and trifluoroacetyl group; sulfonyl protective groups such as a p-toluenesulfonyl group, and methanesulfonyl group; and alkyl protective groups such as an allyl group, benzyl group, and benzhydryl group. Preferably a tert-butoxycarbonyl group, benzyloxycarbonyl group, benzoyl group, and benzyl group are used, because the protective groups can be readily deprotected and the resultant products can be readily extracted from the aqueous reaction mixture with an organic solvent.

The amino group-protecting agent used in the step of protecting the amino group is not limited. Examples of the amino group-protecting agent include carbonic acid diester protecting agents such as di-tert-butyl dicarbonate (DIBOC™); alkoxy carbonyl protecting agents such as chlorocarbonic esters, e.g., benzyl chlorocarbonate, methyl chlorocarbonate, ethyl chlorocarbonate, and allyl chlorocarbonate; acyl chloride protecting agents such as benzoyl chloride, acetyl chloride, and trifluoroacetyl chloride; acyl protecting agents such as acetic anhydride; sulfonylchloride protecting agents such as p-toluenesulfonyl chloride and methanesulfonyl chloride; and alkyl protecting agents such as allyl chloride and benzyl chloride. Preferably di-tert-butyl dicarbonate, benzyl chlorocarbonate, and benzoyl chloride are used, because the protecting group can be readily deprotected and the resultant products can be readily extracted from the aqueous reaction mixture with an organic solvent.

In terms of reaction efficiency, the content of the amino group-protecting agent is preferably 1 molar equivalent or more of the optically active azetidine-2-carboxylic acid (4). Furthermore, the content of the amino group-protecting agent is preferably 3 molar equivalents or less, and more preferably, 1.5 molar equivalents or less of the azetidine-2-carboxylic acid (4) in terms of economical efficiency.

When chlorocarbonic ester protecting agents, acyl chloride protecting agents, and di-tert-butyl carbonate are used as the amino group-protecting agents, examples of the reaction solvents include water, toluene, ethyl acetate, tetrahydrofuran, and a mixture thereof. When sulfonylchloride protecting agents are used as the amino group-protecting agents, examples of the reaction solvents include organic solvents such as toluene, ethyl acetate, tetrahydrofuran, and a mixture thereof.

In terms of reaction efficiency, the step of protecting the amino group is performed in the presence of a base. The base is not limited and examples include inorganic bases such as sodium carbonate, sodium hydrogen carbonate, sodium hydroxide, and potassium hydroxide; and organic bases such as triethylamine, pyridine, and N-methylmorpholine. In terms of reaction efficiency, the base content is preferably 1 molar equivalent or more of the amino group-protecting agent. Furthermore, the base content is preferably 10 molar equivalents or less, and more preferably, 3 molar equivalents or less of the amino group-protecting agent.

Although the temperature during the step of protecting the amino group is not limited, the reaction temperature ranges from the solidifying point to the boiling point of the reaction solvent and is preferably 0°C or more, and more preferably, 20°C or more. Furthermore, the reaction temperature is preferably 100°C or less, and more preferably, 70°C or less. In terms of economical efficiency, the reaction time is preferably 20 hours or less, and more preferably, 10 hours or less. In order to achieve a high yield, the reaction time is preferably one hour or more, and more preferably, two hours or more.

After the completion of the protection of the amino group, hydrochloric acid or an aqueous ammonium chloride solution, for example, is added to the reaction mixture to stop the reaction in a weakly acidic solution. The target substance is extracted with a solvent such as ethyl acetate, diethyl ether, and toluene. If necessary, the extract is washed with, for example, a saturated brine solution. If necessary, the resultant mixture is dried with a drying agent such as sodium sulfate or magnesium sulfate, and is then filtered to remove the drying agent. The mixture is then concentrated. The optically active N-protected azetidine-2-carboxylic acid is separated by any standard method, for example, crystallization or column chromatography. If the resultant optically active N-protected azetidine-2-carboxylic acid does not have sufficiently high optical purity, the optical purity is enhanced by, for example, recrystallization.

### Best Mode for Carrying Out the Invention

The present invention will now be described in detail with reference to the following examples, which do not serve to limit the scope of the present invention. The optical purity is determined by high performance liquid chromatography using a chiral column (Chiral Column OD-R (DAICEL CHEMICAL INDUSTRIES, LTD.)).

### EXAMPLE 1

Dioxane (150 mL) was added to (S)-4-phthalimido-2-hydroxybutyric acid (50 g) in a nitrogen atmosphere. Thionyl chloride (35 mL) was added to the mixture with stirring, and the mixture was stirred at 40°C for one hour. Benzyltriethylammonium chloride (9 g) was then added to the mixture and further stirred at 30°C for 16 hours to produce a solution of dioxane and (R)-4-phthalimido-2-chlorobutyryl chloride. A small amount of the solution was sampled to identify the molecular structure by nuclear magnetic resonance (NMR). The analytical data was as follows:
¹H-NMR (CDCl₃): δ 2.34-2.39 (m,1H), 2.59-2.64 (m,1H), 3.90-3.95 (m,2H), 4.61-4.64 (dd,1H), 7.73-7.77 (m, 2H), 7.84-7.89 (m,2H)

### EXAMPLE 2

The solution of dioxane and (R)-4-phthalimido-2-chlorobutyryl chloride prepared in Example 1 was spontaneously cooled to room temperature. Water (10 mL) was then added to the solution with stirring. The solution was placed in an ice bath and then an aqueous sodium hydroxide solution (400 g/L) (77 mL) was added in order to adjust the pH of the mixture to 2.0. The organic solvent in the mixture was removed under reduced pressure. The resultant mixture was extracted with ethyl acetate to recover a solution of ethyl acetate and (R)-4-phthalimido-2-chlorobutyric acid. A small amount of the solution was sampled to determine the optical purity. The optical purity of the sample was 96.2 %e.e. (enantiomeric excess). The solution of ethyl acetate and (R)-4-phthalimido-2-chlorobutyric acid was concentrated under reduced pressure into about 100 g. Toluene (150 mL) was then added to the solution and the mixture was concentrated under reduced pressure into about 150 g. Furthermore, hexane (170 mL) was added to the resultant solution at 45°C. The solution was gradually cooled to 10°C. The resultant white crystals were filtered and the crystals were dried overnight under reduced pressure with a vacuum pump to recover (R)-4-phthalimido-2-chlorobutyric acid (43 g) (yield 83%, optical purity 100 %e.e.). A small amount of the solution was sampled to identify the molecular structure by NMR. The analytical data was as follows:
¹H-NMR (CDCl₃): δ 2.26-2.35 (m,1H), 2.45-2.53 (m,1H), 3.85-3.96 (m,2H), 4.35-4.39 (dd,1H), 7.72-7.74 (m,2H), 7.85-7.87 (m, 2H)

### EXAMPLE 3

Methanol (40mL) was added to the (R)-4-phthalimido-2-chlorobutyric acid (5 g) and the mixture was stirred. To the mixture 80% hydrazine hydrate (2.3 g) was added with stirring, and the mixture was stirred at 40°C overnight. Water (30 mL) was then added to the mixture with stirring, and 47% sulfuric acid (13 mL) was added. The mixture was stirred at room temperature for 4 hours and the precipitate was filtered out. The filtrate was concentrated under reduced pressure to recover an aqueous solution of (R)-4-amino-2-chlorobutyric acid. A small amount of the solution was sampled to identify the molecular structure by NMR. The analytical data was as follows:
¹H-NMR (D₂O): δ 2.15-2.45 (m,2H), 3.19 (t,2H), 4.45 (t, 1H)

The solution was then placed in an ice bath and an aqueous sodium hydroxide solution (400 g/L) was added to the solution in order to adjust the pH of the solution to 2.0. Water was added to the solution to obtain about 130 g of solution. The resultant solution was heated to about 90°C with stirring. Magnesium hydroxide (1.0 g) was added to the solution and the solution was stirred for 5 hours to produce an aqueous solution of (S)-azetidine-2-carboxylic acid. A small amount of the solution was sampled to identify the molecular structure by NMR. The analytical data was as follows:
¹H-NMR (CD₃OD): δ 2.15 (m,1H), 2.58 (m,1H), 3.90 (m,1H), 4.02 (q,1H), 4.60 (t,1H)

The solution was spontaneously cooled to room temperature. Sodium carbonate (2.1 g) and DIBOC™ (4.3 g) were added with stirring and the mixture was further stirred overnight. Hydrochloric acid (6N) was added to the solution in order to adjust the pH of the solution to 2.0. The resultant solution was extracted with ethyl acetate three times. The resultant organic solution was washed with a saturated brine solution and dried with sodium sulfate. The solvent in the mixture was then removed to recover (S)-N-(tert-butoxycarbonyl)azetidine-2-carboxylic acid (2.1 g) (yield 55%, optical purity 89.3 %e.e.). A small amount of the solution was sampled to identify the molecular structure by NMR. The analytical data was as follows:
¹H-NMR (CDCl₃): δ 1.48 (s,9H), 2.40-2.60 (bs,2H), 3.80-4.00 (bs,2H), 4.80 (t,1H)

### EXAMPLE 4

Dioxane (3 mL) was added to (S)-4-phthalimido-2-hydroxybutyric acid (1.0 g) in a nitrogen atmosphere. Thionyl chloride (2.5 g) was added to the mixture with stirring, and the mixture was stirred at 40°C for one hour. Pyridine (0.06 g) was then added to the mixture and further stirred at 40°C for 15 hours to produce a solution of dioxane and (R)-4-phthalimido-2-chlorobutyryl chloride. The solution was placed in an ice bath and then water (5 mL) was added with stirring. The solution was extracted with ethyl acetate at room temperature. The resultant organic solution was washed with a brine solution and was dried with mirabilite. The resultant solution containing ethyl acetate was concentrated under reduced pressure to recover (R)-4-phthalimido-2-chlorobutyric acid. Methanol (9 mL) was added to the compound. To the mixture 80% hydrazine hydrate (0.5 g) was added with stirring, and the mixture was stirred at 40°C overnight. Water (6 mL) was then added to the solution with stirring and 47% sulfuric acid (3 mL) was added to the solution. The mixture was stirred at room temperature for three hours and the precipitate was filtered. The filtrate was concentrated under reduced pressure to produce an aqueous solution of (R)-4-amino-2-chlorobutyric acid. The solution was then placed in an ice bath and an aqueous sodium hydroxide solution (400 g/L) was added to the solution in order to adjust the pH of the solution to 2.0. Water was added to the solution to obtain about 30 g of solution. The resultant solution was heated to about 80°C with stirring. Magnesium hydroxide (0.20 g) was added to the solution and the solution was stirred for 10 hours to produce an aqueous solution of (S)-azetidine-2-carboxylic acid. The solution was spontaneously cooled to room temperature. Sodium carbonate (0.43 g) and DIBOC™ (0.90 g) were added with stirring and the mixture was further stirred overnight. Hydrochloric acid (6N) was added to the solution in order to adjust the pH of the solution to 2.0. The resultant mixture was extracted with ethyl acetate three times. The resultant organic solution was washed with a saturated brine solution and dried with sodium sulfate. The solvent in the mixture was then removed to recover (S)-N-(tert-butoxycarbonyl)azetidine-2-carboxylic acid (0.32 g) (yield 41%, optical purity 87.1 %e.e.).

### Industrial Applicability

According to the present invention, optically active azetidine-2-carboxylic acid, which is an important material for medicines, can be efficiently, readily, and commercially produced by halogenation of an optically active N-protected 4-amino-2-hydroxybutyric acid following inversion of the configuration, hydrolyzing the product, deprotecting the amino group, and then cyclizing the product.

Furthermore, according to the present invention, an optically active N-protected 4-amino-2-halobutyryl halide represented by general formula (2) is a useful compound for producing an optically active azetidine-2-carboxylic acid, which is an important material for medicines.

## Claims

1. A process for producing an optically active N-protected 4-amino-2-halobutyryl halide represented by general formula (2): 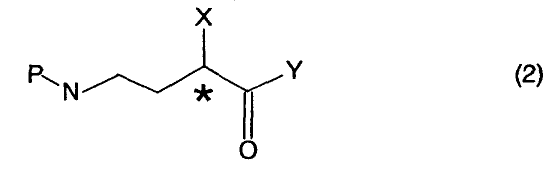 (wherein P represents a protective group for the primary amino group; * indicates that the carbon atom is asymmetric; and each of X and Y independently represents a halogen atom), the process comprising halogenating an optically active N-protected 4-amino-2-hydroxybutyric acid following inversion of the configuration, the optically active N-protected 4-amino-2-hydroxybutyric acid being represented by general formula (1): 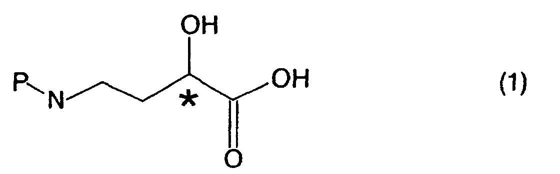 (wherein P and * are as defined above).

2. A process for producing an optically active N-protected 4-amino-2-halobutyric acid represented by general formula (3): 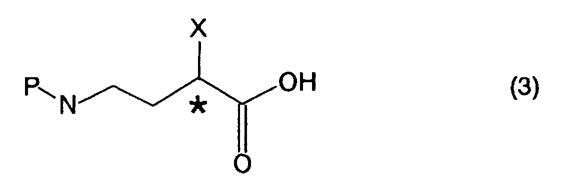 (wherein P represents a protective group for the primary amino group; * indicates that the carbon atom is asymmetric; and X represents a halogen atom), the process comprising hydrolyzing an optically active N-protected 4-amino-2-halobutyryl halide represented by general formula (2): 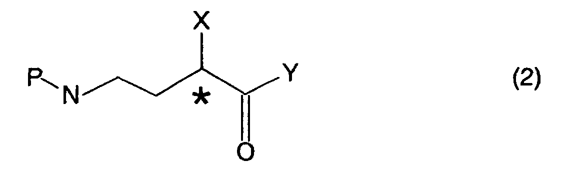 (wherein P and * are as defined above; each of X and Y independently represents a halogen atom).

3. A process for producing an optically active N-protected 4-amino-2-halobutyric acid represented by general formula (3): 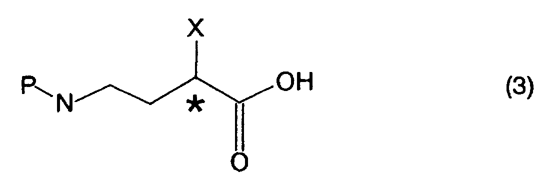 (wherein P represents a protective group for the primary amino group; * indicates that the carbon atom is asymmetric; and X represents a halogen atom), the process comprising the steps of:
halogenating an optically active N-protected 4-amino-2-hydroxybutyric acid following inversion of the configuration, the optically active N-protected 4-amino-2-hydroxybutyric acid being represented by general formula (1): 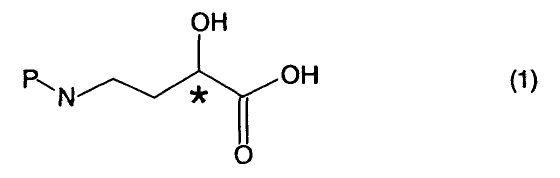 (wherein P and * are as defined above) to produce an optically active N-protected 4-amino-2-halobutyryl halide represented by general formula (2): 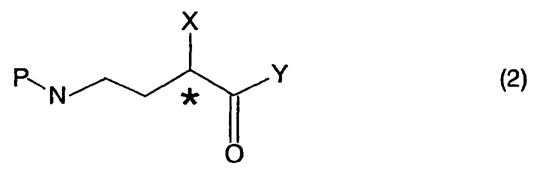 (wherein P and * are as defined above; and each of X and Y independently represents a halogen atom); and
hydrolyzing the optically active N-protected 4-amino-2-halobutyryl halide represented by general formula (2).

4. A process for producing an optically active azetidine-2-carboxylic acid represented by general formula (4): 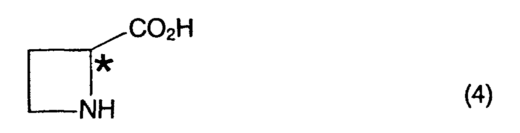 (wherein * indicates that the carbon atom is asymmetric), the process comprising the steps of:
halogenating an optically active N-protected 4-amino-2-hydroxybutyric acid following inversion of the configuration, the optically active N-protected 4-amino-2-hydroxybutyric acid being represented by general formula (1): 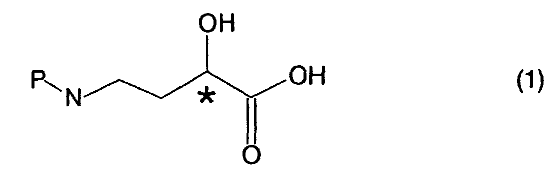 (wherein P represents a protective group for the primary amino group; and * is as defined above) to produce an optically active N-protected 4-amino-2-halobutyryl halide represented by general formula (2): 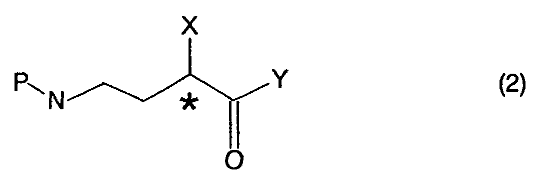 (wherein P and * are as defined above; and each of X and Y independently represents a halogen atom);
hydrolyzing the optically active N-protected 4-amino-2-halobutyryl halide represented by general formula (2) to produce an optically active N-protected 4-amino-2-halobutyric acid represented by general formula (3): 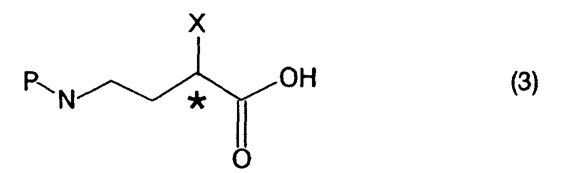 (wherein P, *, and X are as defined above);
deprotecting the amino group of the optically active N-protected 4-amino-2-halobutyric acid represented by general formula (3); and
cyclizing the deprotected product in an alkaline aqueous solution.

5. A process for producing an optically active N-protected azetidine-2-carboxylic acid represented by general formula (5): 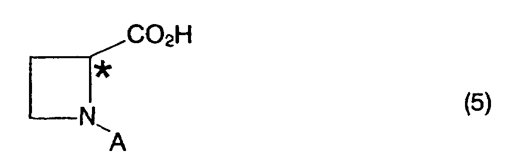 (wherein A represents a protective group for the secondary amino group; and * indicates that the carbon atom is asymmetric), the process comprising the steps of:
halogenating an optically active N-protected 4-amino-2-hydroxybutyric acid following inversion of the configuration, the optically active N-protected 4-amino-2-hydroxybutyric acid being represented by general formula (1): 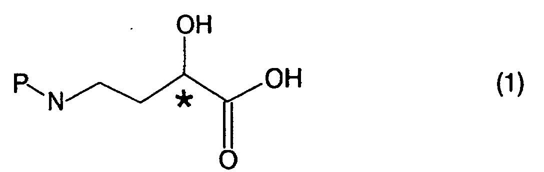 (wherein P represents a protecting group for the primary amino group; and * is as defined above) to produce an optically active N-protected 4-amino-2-halobutyryl halide represented by general formula (2): 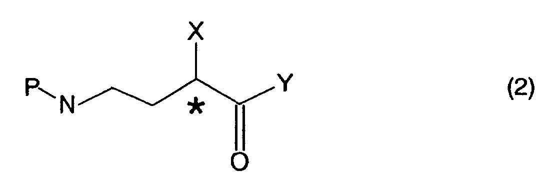 (wherein P and * are as defined above; and each of X and Y independently represents a halogen atom);
hydrolyzing the optically active N-protected 4-amino-2-halobutyryl halide represented by general formula (2) to produce an optically active N-protected 4-amino-2-halobutyric acid represented by general formula (3): 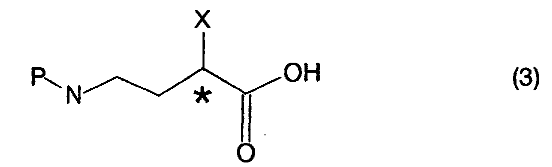 (wherein P, *, and X are as defined above);
deprotecting the amino group of the optically active N-protected 4-amino-2-halobutyric acid represented by general formula (3);
cyclizing the deprotected product in an alkaline aqueous solution to produce an optically active azetidine-2-carboxylic acid represented by general formula (4): 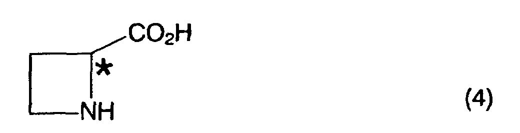 (wherein * is as defined above); and
reacting the optically active azetidine-2-carboxylic acid represented by general formula (4) with an amino group-protecting agent.

6. The process according to any one of claim 1 to claim 5, wherein X and Y are each independently a chlorine atom or a bromine atom.

7. The process according to any one of claim 1 to claim 6, wherein P representing the protective group for the primary amino group is a phthalimido group or an alkoxy carbonyl group.

8. The process according to claim 7, wherein the alkoxy carbonyl group is a benzyloxycarbonyl group, a methoxycarbonyl group, an ethoxycarbonyl group, or a tert-butoxycarbonyl group.

9. The process according to claim 5 or 6, wherein A representing the protective group for the secondary amino group is an alkoxy carbonyl group.

10. The process according to claim 9, wherein the alkoxy carbonyl group is a tert-butoxycarbonyl group.

11. The process according to claim 1, 3, 4, 5, 6, 7, 8, 9, or 10, wherein thionyl chloride is used as a halogenating agent in the step of halogenating the optically active N-protected 4-amino-2-hydroxybutyric acid represented by general formula (1).

12. The process according to claim 1, 3, 4, 5, 6, 7, 8, 9, 10, or 11, wherein an ammonia salt, a compound having a primary amino group, a compound having a secondary amino group, a compound having a tertiary amino group, or a compound having a quaternary amino group is added in the step of halogenating the optically active N-protected 4-amino-2-hydroxybutyric acid represented by general formula (1).

13. The process according to claim 1, 3, 4, 5, 6, 7, 8, 9, 10, or 11, wherein an ammonia salt, an amine, an imine, an amide, an imide, urea, or salts thereof is added in the step of halogenating the optically active N-protected 4-amino-2-hydroxybutyric acid represented by general formula (1).

14. The process according to claim 1, 3, 4, 5, 6, 7, 8, 9, 10, or 11, wherein pyridine, triethylamine, imidazole, N,N-dimethylformamide, or benzyltriethylammonium chloride is added in the step of halogenating the optically active N-protected 4-amino-2-hydroxybutyric acid represented by general formula (1).

15. An optically active N-protected 4-amino-2-halobutyryl halide represented by general formula (2): 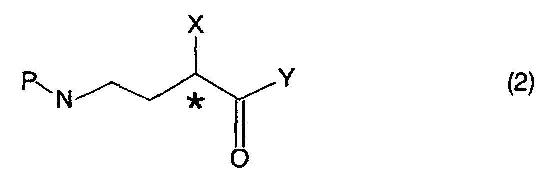 (wherein P represents a protective group for the primary amino group; * indicates that the carbon atom is asymmetric; and each of X and Y independently represents a halogen atom).

16. The optically active N-protected 4-amino-2-halobutyryl halide according to claim 15, wherein X and Y are each independently a chlorine atom or a bromine atom.

17. The optically active N-protected 4-amino-2-halobutyryl halide according to claim 15 or 16, wherein P representing a protective group for the primary amino group is a phthalimido group or an alkoxy carbonyl group.

18. The optically active N-protected 4-amino-2-halobutyryl halide according to claim 17, wherein the alkoxy carbonyl group is a benzyloxycarbonyl group, a methoxycarbonyl group, an ethoxycarbonyl group, or a tert-butoxycarbonyl group.
